(19) Europäisches Patentamt / European Patent Office / Office européen des brevets

(11) **EP 2 962 623 A1**

(12) **EUROPEAN PATENT APPLICATION**
published in accordance with Art. 153(4) EPC

(43) Date of publication:
**06.01.2016 Bulletin 2016/01**

(51) Int Cl.:
*A61B 1/04* (2006.01)    *A61B 1/00* (2006.01)
*A61B 1/06* (2006.01)    *H04N 7/18* (2006.01)

(21) Application number: **14757268.9**

(22) Date of filing: **17.01.2014**

(86) International application number:
**PCT/JP2014/050772**

(87) International publication number:
**WO 2014/132695 (04.09.2014 Gazette 2014/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **27.02.2013   JP 2013037294**

(71) Applicant: **Olympus Corporation**
**Shibuya-ku**
**Tokyo 151-0072 (JP)**

(72) Inventors:
• **HIROTA, Masashi**
  **Tokyo 151-0072 (JP)**
• **KANDA, Yamato**
  **Tokyo 151-0072 (JP)**

(74) Representative: **Gunzelmann, Rainer**
**Wuesthoff & Wuesthoff**
**Patentanwälte PartG mbB**
**Schweigerstraße 2**
**81541 München (DE)**

(54) **IMAGE PROCESSING DEVICE, IMAGE PROCESSING METHOD, AND IMAGE PROCESSING PROGRAM**

(57)    Provided is an image processing apparatus or the like which can accurately extract a blood vessel in a specified depth from an image acquired by an endoscope and can highlight the blood vessel. An image processing apparatus 1 includes a narrow-band image acquisition unit 101 to acquire at least three narrow-band images with different center wavelengths in an image processing apparatus to process an image acquired by capturing of a living body, a depth feature data calculation unit 102 to calculate depth feature data, which is feature data correlated to a depth of a blood vessel in the living body, based on a difference, between the narrow-band images different from one another, in variation of signal intensity due to an absorption variation of light with which the living body is irradiated, and an enhanced image creation unit 103 to create, based on the depth feature data, an image in which a blood vessel is highlighted according to a depth of the blood vessel.

FIG.1

EP 2 962 623 A1

**Description**

Field

The present invention relates to an image processing apparatus, an image processing method, and an image processing program for performing image processing on an image acquired by an endoscope which observes inside of a lumen of a living body.

Background

In recent years, endoscopes have been widely used as a medical observation apparatus which can observe a lumen of a living body in a non-invasive manner. As a light source of an endoscope, a white light source such as a xenon lamp is usually used. By combining the light source and a rotary filter in which a red filter, a green filter, and a blue filter to respectively pass pieces of light having wavelength bands of red light (R), green light (G), and blue light (B), a band of white light emitted by the light source is narrowed and the inside of a lumen is irradiated with the white light. From an image acquired accordingly, a rough shape or a state of a mucous membrane in a lumen, or existence of a polyp can be observed.

In a case of performing observation by using white light, there has been a problem that visibility of a blood vessel in a surface layer or a deep layer of a mucous membrane is low and clear observation is difficult. In order to cope with such a problem, in Patent Literature 1, a technique to highlight or control a blood vessel region in a specified depth is disclosed. More specifically, in Patent Literature 1, a narrow-band signal (narrow-band image data) and a wide-band signal (wide-band image signal) are acquired by capturing of a lumen. A depth of a blood vessel is estimated based on a luminance ratio between these signals. When it is determined that the blood vessel is in a surface layer, contrast in the blood vessel region is changed to display an image.

Citation List

Patent Literature

Patent Literature 1: Japanese Laid-open Patent Publication No. 2011-98088

Summary

Technical Problem

However, a ratio between a narrow-band signal and a wide-band signal in an actual endoscopic image varies due to an influence of an element other than a depth of a blood vessel, such as intensity of emitted light, an individual difference of a living body which is a subject, or a thickness of a blood vessel. Thus, when a depth of a blood vessel is estimated based simply on a ratio between a narrow-band signal and a wide-band signal in such a manner described in Patent Literature 1, adequate estimation accuracy is not acquired and it is difficult to highlight a blood vessel in a depth intended by a user.

The present invention has been made in view of the forgoing, and an object of the invention is to provide an image processing apparatus, an image processing method, and an image processing program which can accurately extract a blood vessel in a specified depth from an image acquired by an endoscope and can highlight the blood vessel.

Solution to Problem

To solve the problem described above and to achieve the object, an image processing apparatus according to the invention processes an image acquired by imaging a living body. The image processing apparatus includes: a narrow-band image acquisition unit configured to acquire at least three narrow-band images with different center wavelengths from one another; a depth feature data calculation unit configured to calculate depth feature data which is feature data correlated to a depth of a blood vessel in the living body based on a difference, between the narrow-band images different from one another, in variation of signal intensity due to an absorption variation of light with which the living body is irradiated; and an enhanced image creation unit configured to create, based on the depth feature data, an image in which the blood vessel is highlighted according to the depth of the blood vessel. The depth feature data calculation unit includes: a normalized feature data calculation unit configured to calculate pieces of normalized feature data by normalizing a value corresponding to signal intensity of each pixel in the at least three narrow-band images; and a relative feature data calculation unit configured to calculate relative feature data indicating a relative relationship in intensity between the pieces of normalized feature data in the narrow-band images different from one another.

An image processing method according to the invention is executed by an image processing apparatus for processing an image acquired by imaging a living body. The method includes: a narrow-band image acquisition step of acquiring at least three narrow-band images with different center wavelengths from one another; a depth feature data calculation step of calculating depth feature data which is feature data correlated to a depth of a blood vessel in the living body based on a difference, between the narrow-band images different from one another, in variation of signal intensity due to an absorption variation of light with which the living body is irradiated; and an enhanced image creation step of creating, based on the depth feature data, an image in which the blood vessel is highlighted according to the depth of the blood vessel. The depth feature data calculation step includes: a normalized feature data calculation step of calculating pieces of normalized feature data by normalizing a value corresponding to signal intensity of each pixel in the at least three narrow-band images; and a relative feature data calculation step of calculating relative feature data indicating a relative relationship in intensity between the pieces of normalized feature data in the narrow-band images different from one another.

An image processing program according to the invention causes an image processing apparatus for processing an image acquired by imaging a living body, to execute: a narrow-band image acquisition step of acquiring at least three narrow-band images with different center wavelengths from one another; a depth feature data calculation step of calculating depth feature data which is feature data correlated to a depth of a blood vessel in the living body based on a difference, between the narrow-band images different from one another, in variation of signal intensity due to an absorption variation of light with which the living body is irradiated; and an enhanced image creation step of creating, based on the depth feature data, an image in which the blood vessel is highlighted according to the depth of the blood vessel. The depth feature data calculation step includes: a normalized feature data calculation step of calculating pieces of normalized feature data by normalizing a value corresponding to signal intensity of each pixel in the at least three narrow-band images; and a relative feature data calculation step of calculating relative feature data indicating a relative relationship in intensity between the pieces of normalized feature data in the narrow-band images different from one another.

Advantageous Effects of Invention

According to the present invention, based on a difference in variation of signal intensity due to absorption variation of light with which a living body is irradiated, depth feature data which is feature data correlated to a depth of a blood vessel of the living body is calculated. Also, based on the depth feature data, an image in which a blood vessel is highlighted is created according to a depth of the blood vessel. Accordingly, it is possible to accurately extract a blood vessel in a depth intended by a user and to highlight the blood vessel.

Brief Description of Drawings

FIG. 1 is a block diagram illustrating a configuration of an image processing apparatus according to a first embodiment of the present invention.
FIG. 2 is a flowchart illustrating an operation of the image processing apparatus illustrated in FIG. 1.
FIG. 3 is a flowchart illustrating processing executed by a normalized feature data calculation unit illustrated in FIG. 1.
FIG. 4 is a diagram illustrating a relationship between signal intensity of a pixel indicating a blood vessel in a narrow-band image and a depth of the blood vessel.
FIG. 5 is a flowchart illustrating processing executed by an enhanced image creation unit illustrated in FIG. 1.
FIG. 6 is a block diagram illustrating a configuration of a normalized feature data calculation unit included in an image processing apparatus according to a modification example of the first embodiment of the present invention.
FIG. 7 is a diagram illustrating a relationship between signal intensity of a pixel indicating a blood vessel in a narrow-band image and a depth of the blood vessel when the blood vessel is thick.
FIG. 8 is a diagram illustrating a relationship between signal intensity of a pixel indicating a blood vessel in a narrow-band image and a depth of the blood vessel when the blood vessel is thin.
FIG. 9 is a flowchart illustrating processing executed by the normalized feature data calculation unit illustrated in FIG. 6.
FIG. 10 is a block diagram illustrating a configuration of an image processing apparatus according to a second embodiment of the present invention.
FIG. 11 is a flowchart illustrating an operation of the image processing apparatus illustrated in FIG. 10.
FIG. 12 is a flowchart illustrating processing executed by a normalized feature data calculation unit illustrated in FIG. 10.

Description of Embodiments

An image processing apparatus, an image processing method, and an image processing program according to some embodiments of the present invention will be described below with reference to the drawings. Note that the present invention is not limited to the embodiments. The same reference signs are used to designate the same elements throughout the drawings.

(First Embodiment)

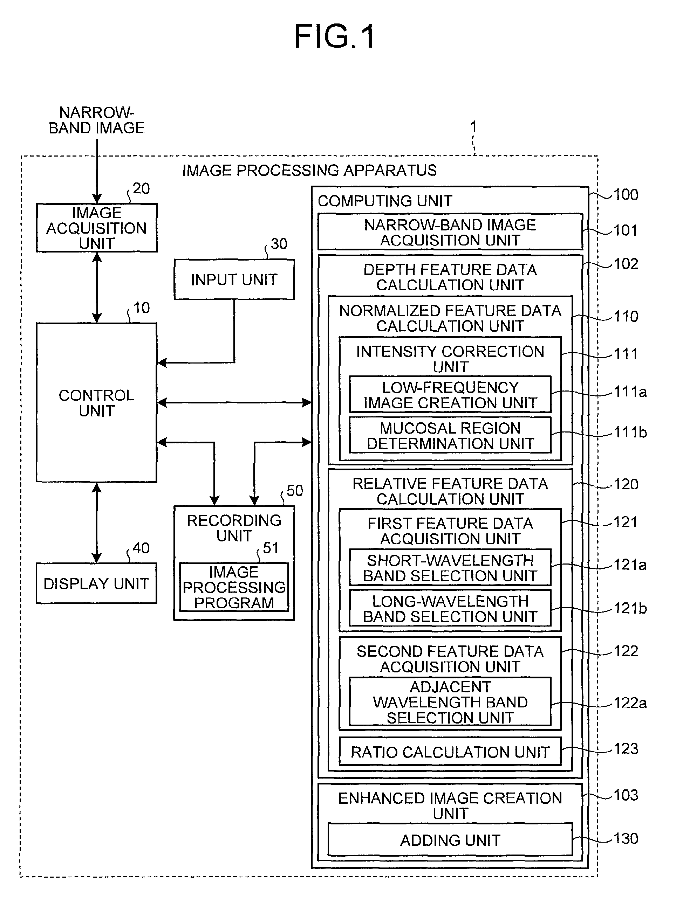

FIG. 1 is a block diagram illustrating an image processing apparatus according to the first embodiment of the present invention. The image processing apparatus 1 according to the first embodiment is an apparatus to estimate a depth of a blood vessel in an image by using at least three narrow-band images having different center wavelengths and to perform image processing of creating an intraluminal image in which a blood vessel is highlighted with different colors according to an a depth. Note that in the following description, a narrow-band image acquired by imaging the inside of a lumen of a living body with an endoscope or a capsule endoscope is a target of processing. However, an image acquired by an observation apparatus other than the endoscope and the capsule endoscope may be used as a target of processing.
As an example of an acquisition method of a narrow-band image with an endoscope, there is a method of using an LED which emits light having a plurality of wavelength peaks in narrow bands. For example, an LED to emit light having peaks at wavelengths of 415 nm, 540 nm, and 600 nm and an LED to emit light having peaks at wavelength of 460 nm, 540 nm, and 630 nm are provided in an endoscope. These LEDs are made to emit light alternately and the inside of the living body is irradiated. Then, a red (R) component, a green (G) component, and a blue (B) component of reflection light from the living body are acquired by a color imaging element. Accordingly, it is possible to acquire five kinds of narrow-band images respectively including wavelength components of 415 nm, 460 nm, 540 nm, 600 nm, and 630 nm.
Alternatively, as a different example of an acquisition method of a narrow-band image, there is a method to arrange a narrow-band filter in front of a white light source such as a xenon lamp and to serially irradiate a living body with light a band of which is narrowed by the narrow-band filter or a method to serially drive a plurality of laser diodes which respectively emit pieces of narrow-band light having different center wavelengths. Moreover, a narrow-band image may be acquired by irradiating a living body with white light and by making reflection light from the living body incident to an imaging element through a narrow-band filter.
As illustrated in FIG. 1, the image processing apparatus 1 includes a control unit 10 to control a whole operation of the image processing apparatus 1, an image acquisition unit 20 to acquire image data corresponding to a narrow-band image captured by an endoscope, an input unit 30 to generate an input signal according to operation from the outside, a display unit 40 to perform various kinds of displaying, a recording unit 50 to store image data acquired by the image acquisition unit 20 or various programs, and a computing unit 100 to execute predetermined image processing on image data.
The control unit 10 is realized by hardware such as a CPU. By reading various programs recoded in the recording unit 50, the control unit 10 transfers an instruction or data to each part included in the image processing apparatus 1 according to image data input from the image acquisition unit 20, an operation signal input from the input unit 30, or the like and controls a whole operation of the image processing apparatus 1 integrally.
The image acquisition unit 20 is configured arbitrarily according to a form of a system including an endoscope. For example, when a portable recording medium is used for passing image data to a capsule endoscope, the image acquisition unit 20 includes a reader apparatus to which the recording medium is mounted in a detachable manner and which reads image data of a recorded image. Also, in a case of providing a server to save image data of an image captured by an endoscope, the image acquisition unit 20 includes a communication apparatus or the like connected to the server and performs data communication with the server to acquire image data. Alternatively, the image acquisition unit 20 may include an interface or the like to input an image signal from an endoscope through a cable.
The input unit 30 is realized, for example, by an input device such as a keyboard, a mouse, a touch panel, or various switches and outputs, to the control unit 10, an input signal generated according to operation on the input device from the outside.
The display unit 40 is realized, for example, by a display device such an LCD or an EL display and displays various screens including an intraluminal image under control by the control unit 10.
The recording unit 50 is realized, for example, by various IC memories including a ROM such as a flash memory capable of update recording, or a RAM, by a hard disk which is built in or which is connected via a data communication terminal, or by an information recording apparatus such as a CD-ROM and a reading apparatus thereof. In addition to the image data acquired by the image acquisition unit 20, the recording unit 50 stores a program to operate the image processing apparatus 1 and to cause the image processing apparatus 1 to execute various functions, data used in execution of the program, or the like. More specifically, the recording unit 50 stores, for example, an image processing program 51 to cause the image processing apparatus 1 to execute image processing to create an image, in which a blood vessel in a living body is highlighted in a color corresponding to a depth from a surface layer, based on a plurality of narrow-band images acquired by an endoscope.

The computing unit 100 is realized by hardware such as a CPU. By reading the image processing program 51, the computing unit 100 performs image processing on a plurality of narrow-band images and creates an image in which a blood vessel in a living body is highlighted in a color corresponding to a depth from a surface layer.

Next, a configuration of the computing unit 100 will be described. As illustrated in FIG. 1, the computing unit 100 includes a narrow-band image acquisition unit 101 to read image data of at least three narrow-band images from the recording unit 50, a depth feature data calculation unit 102 to calculate feature data correlated to a depth of a blood vessel in a living body based on the narrow-band images acquired by the narrow-band image acquisition unit 101, and an enhanced image creation unit 103 to create, based on the feature data, an image in which a blood vessel is highlighted in a color corresponding to a depth of the blood vessel.

The narrow-band image acquisition unit 101 acquires at least three narrow-band images captured with pieces of narrow-band light having different center wavelengths. Preferably, at least narrow-band images respectively including an R component, a G component, and a B component are acquired.

Based on a difference, between the narrow-band images different from one another, in variation of signal intensity due to an absorption variation of light with which a living body is irradiated, the depth feature data calculation unit 102 calculates feature data correlated to a depth of a blood vessel in the living body (hereinafter, referred to as depth feature data). More specifically, the depth feature data calculation unit 102 includes a normalized feature data calculation unit 110 to normalize signal intensity of each pixel in narrow-band images acquired by the narrow-band image acquisition unit 101 and a relative feature data calculation unit 120 to calculate relative feature data, which is feature data indicating relative signal intensity of each pixel in two narrow-band images, based on the normalized signal intensity (hereinafter, also referred to as normalized signal intensity).

Here, the normalized feature data calculation unit 110 includes an intensity correction unit 111 to correct, with signal intensity in a mucosal region as a reference, signal intensity of each pixel in the narrow-band images acquired by the narrow-band image acquisition unit 101. The intensity correction unit 111 includes a low-frequency image creation unit 111a and a mucosal region determination unit 111b. The low-frequency image creation unit 111a calculates a low-frequency image in which a low-frequency component in a spatial frequency component included in each narrow-band image is a pixel value. Also, based on each narrow-band image and the low-frequency image, the mucosal region determination unit 111b identifies a mucosal region in each narrow-band image.

The relative feature data calculation unit 120 includes a first feature data acquisition unit 121, a second feature data acquisition unit 122, and a ratio calculation unit 123. Here, the first feature data acquisition unit 121 selects one narrow-band image (first narrow-band image) from the narrow-band images acquired by the narrow-band image acquisition unit 101 and acquires normalized signal intensity in the selected narrow-band image as first feature data. The first feature data acquisition unit 121 includes a short-wavelength band selection unit 121a for selecting a narrow-band image including a wavelength component with a relatively short wavelength (such as B component or G component) from the narrow-band images acquired by the narrow-band image acquisition unit 101, and a long-wavelength band selection unit 121b for selecting a narrow-band image including a wavelength component with relatively short wavelength (such as R component or G component).

Based on a wavelength component of the narrow-band image selected by the first feature data acquisition unit 121, the second feature data acquisition unit 122 selects a different narrow-band image (second narrow-band image) from the narrow-band images acquired by the narrow-band image acquisition unit 101 and acquires normalized signal intensity of the narrow-band image as second feature data. More specifically, the second feature data acquisition unit 122 includes an adjacent wavelength band selection unit 122a to select a narrow-band image with a wavelength component a band of which is adjacent to that of the narrow-band image selected by the short-wavelength band selection unit 121a or the long-wavelength band selection unit 121b.

The ratio calculation unit 123 calculates a ratio between the first feature data and the second feature data as feature data indicating relative signal intensity between narrow-band images.

The enhanced image creation unit 103 includes an adding unit 130 for adding narrow-band images to one another. Based on the depth feature data calculated by the depth feature data calculation unit 102, the enhanced image creation unit 103 weights and adds the narrow-band image acquired by the narrow-band image acquisition unit 101 and the narrow-band image corrected by the intensity correction unit 111, and thereby creates an image in which a blood vessel is highlighted in a color corresponding to the depth.

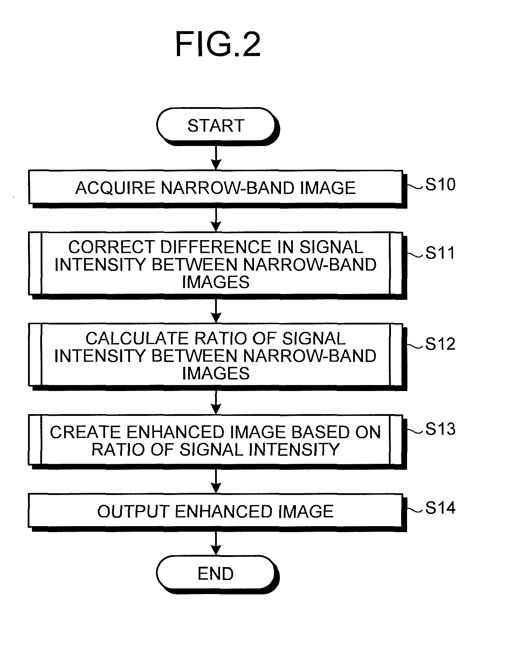

Next, an operation of the image processing apparatus 1 will be described. FIG. 2 is a flowchart illustrating an operation of the image processing apparatus 1.

First, in step S10, the narrow-band image acquisition unit 101 acquires at least three narrow-band images having different center wavelengths. A combination of at least three narrow-band images is not limited to a combination of a red band image, a green band image, and a blue narrow-band image as long as the combination is a combination of images having wavelength bands with different kinds of signal intensity of a pixel with respect to a depth of a blood vessel from a mucosal surface in a living body. In the following description, for example, five narrow-band images respectively having center wavelengths of 415 nm, 460 nm, 540 nm, 600 nm, and 630 nm are acquired.

Then, in next step S11, the normalized feature data calculation unit 110 corrects a difference in signal intensity between the narrow-band images acquired in step S10. In narrow-band images with different center wavelengths, even when the same region is captured, a difference in signal intensity is generated due to a difference in intensity of narrow-band light with which a mucosal surface or the like of a living body is irradiated, spectral reflectivity on an irradiated surface, or the like. Thus, the correction is performed to make it possible to calculate feature data which can be compared in the narrow-band images. Here, absorption of narrow-band light, which has a center wavelength of 630 nm among the above-described five wavelengths, by hemoglobin is significantly low. Thus, it can be considered that signal intensity of each pixel in the narrow-band image with the center wavelength of 630 nm roughly indicates a mucosal surface. Thus, in the first embodiment, with the narrow-band image having the center wavelength of 630 nm as a reference, correction is performed in such a manner that signal intensity of pixels indicating mucosal surfaces in the four other narrow-band images becomes equivalent.

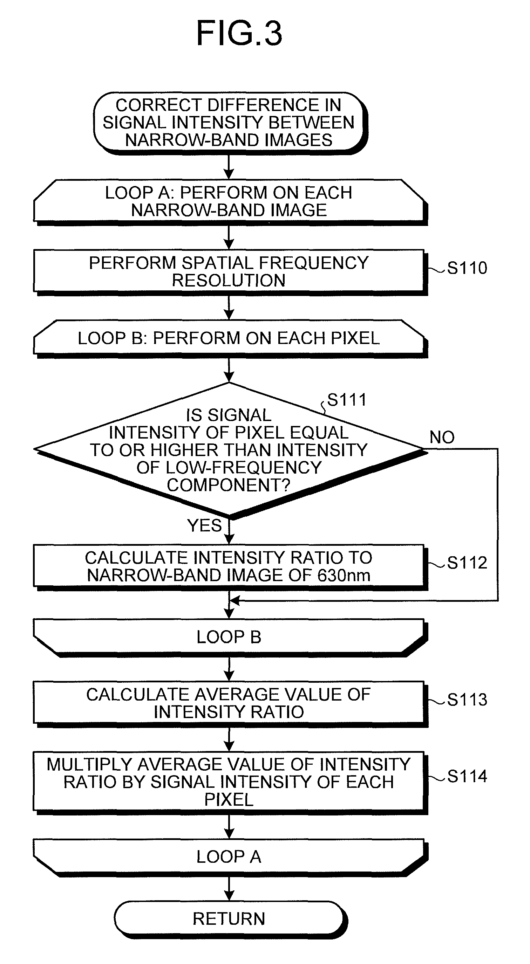

FIG. 3 is a flowchart illustrating processing executed by the normalized feature data calculation unit 110 in step S11. The normalized feature data calculation unit 110 performs processing in a loop A on each narrow-band image other than a reference narrow-band image (narrow-band image of 630 nm in the first embodiment) among the narrow-band images acquired by the narrow-band image acquisition unit 101.

First, in step S110, the low-frequency image creation unit 111a performs spatial frequency resolution on a narrow-band image as a processing target to divide into a plurality of spatial frequency bands, and creates an image (hereinafter, referred to as low-frequency image) having, as a pixel value, intensity of a component in a low-frequency band (low-frequency component). The spatial frequency resolution can be performed, for example, according to Difference Of Gaussian (DOG) (reference: Advanced Communication Media CO., LTD., "Computer Vision and Image Media 2," pp. 8).

Reference will be made below to an outline of processing of creating a low-frequency image according to DOG. First, a smoothed image $L_i$ is calculated by convolution calculation of a narrow-band image and a Gaussian function of a scale $\sigma = \sigma_0$. Here, the sign i is a parameter indicating the number of times of calculation and i = 1 is set as an initial value. Then, by performing a convolution calculation of the smoothed image $L_i$ and a Gaussian function of a scale $\sigma = k^i\sigma_0$, a smoothed image $L_{i+1}$ is calculated. Here, the sign k indicates an increase rate of the Gaussian function. Such processing is repeatedly performed while increment of parameter i is performed. Then, a difference image between arbitrary two smoothed images $L_{i=n}$ and $L_{i=m}$ (n and m are natural number) is acquired. The difference image is an image including a specific frequency component. By arbitrarily selecting parameters n and m of the smoothed images $L_{i=n}$ and $L_{i=m}$ from which a difference image is acquired, a low-frequency image can be acquired.

Then, processing in a loop B is performed on each pixel in the narrow-band images. That is, in step S111, the mucosal region determination unit 111b compares signal intensity of each pixel in the narrow-band images with intensity of a low-frequency component of the pixel acquired by the spatial frequency resolution and determines whether the signal intensity of the pixel is higher than the intensity of the low-frequency component. More specifically, the mucosal region determination unit 111b compares pixel values of pixels corresponding to each other in each narrow-band image and the low-frequency image created in step S110.

In a case where the signal intensity of the pixel is lower than the intensity of the low-frequency component (step S111: No), the intensity correction unit 111 determines that the pixel is not a mucosal surface and proceeds to processing with respect to a next pixel. On the other hand, when the signal intensity of the pixel is higher than the intensity of the low-frequency component (step S111: Yes), the intensity correction unit 111 determines that the pixel is a mucosal surface and calculates a ratio (intensity ratio: $I_{630}/I_\lambda$) to signal intensity of a corresponding pixel in the narrow-band image with a wavelength of 630 nm (step S112). Here, the sign $I_\lambda$ ($\lambda$ = 415 nm, 460 nm, 540 nm, or 600 nm) indicates signal intensity of a pixel being processed in a narrow-band image as a processing target. Also, the sign $I_{630}$ indicates signal intensity of a pixel corresponding to the above-described pixel being processed in the narrow-band image with the wavelength of 630 nm.

When determination of a mucosal surface with respect to all pixels in the narrow-band image as a processing target is over, in next step S113, the normalized feature data calculation unit 110 calculates an average value AVG ($I_{630}/I_\lambda$) of intensity ratios $I_{630}/I_\lambda$ of all pixels which are determined as mucosal surfaces.

Also, in step S114, the normalized feature data calculation unit 110 multiplies the average value AVG ($I_{630}/I_\lambda$) by signal intensity of each pixel in the narrow-band images. Signal intensity $I_\lambda' = I_\lambda \times$ AVG ($I_{630}/I_\lambda$) of each pixel after the multiplication is treated as corrected signal intensity in the following processing.

These steps S110 to S114 are performed on each of the narrow-band images other than the reference narrow-band image. Thus, in these narrow-band images, it is possible to correct a difference in signal intensity due to intensity of narrow-band light, spectral reflectivity, or the like. Then, an operation of the image processing apparatus 1 goes back to a main routine.

Note that in the above description, intensity of a low-frequency component of each pixel is calculated by spatial frequency resolution. However, well-known various methods (such as smoothing filter) other than the spatial frequency resolution may be used.

Also, in the above description, a mucosal surface is identified based on a relative intensity relationship between signal intensity of each pixel in the narrow-band images and a low-frequency component. However, a different method can be used as long as correction can be performed in such a manner that signal intensity on mucosal surfaces becomes equivalent in a plurality of narrow-band images. For example, an average value AVG $(I_{630}/I_\lambda)$ may be calculated by creating a distribution of a ratio of signal intensity (intensity ratio) between each pixel in a narrow-band image as a processing target and a corresponding pixel in a narrow-band image of 630 nm and by calculating a weighted average such that the weight becomes larger as the intensity ratio has relatively higher frequency in the distribution of the intensity ratio.

Also, in the above description, signal intensity of narrow-band images is corrected with a narrow-band image of 630 nm as a reference. However, a narrow-band image other than 630 nm may be used as a reference. For example, in processing in the following stage, in a case where a combination of narrow-band images in which a relative relationship of signal intensity between corresponding pixels is necessary is previously known, correction of the signal intensity may be performed in the combination of the narrow-band images.

In step S12 following step S11, the relative feature data calculation unit 120 calculates a ratio of the signal intensity (intensity ratio), which is corrected in step S11, between the narrow-band images different from one another. The intensity ratio is depth feature data correlated to a depth of a blood vessel in a living body.

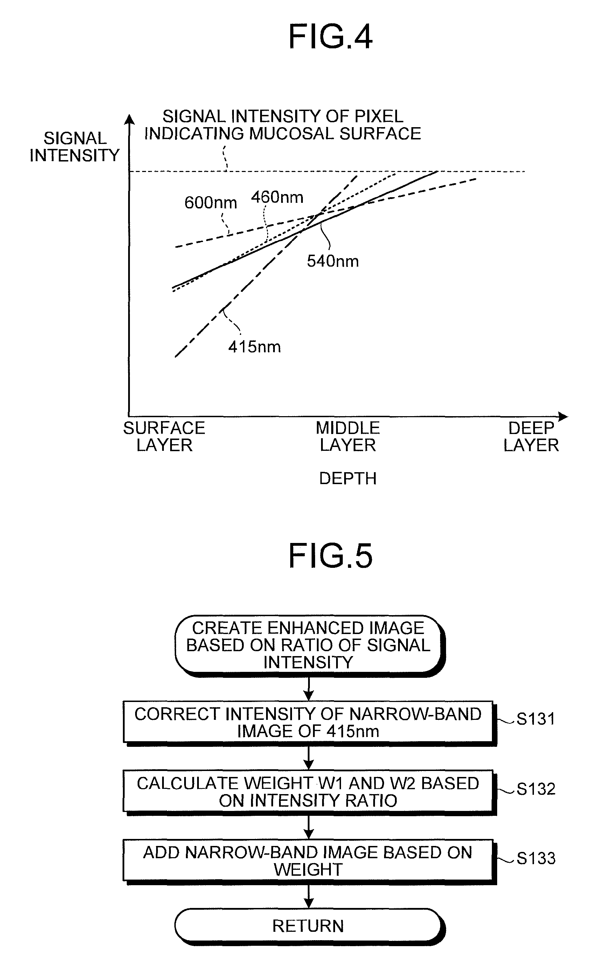

Here, narrow-band light with which a living body is irradiated is scattered less on a mucosal surface and reaches a deeper layer as a wavelength becomes longer. Also, absorption of narrow-band light, which is used in the first embodiment, in hemoglobin is the highest in narrow-band light of 415 nm and becomes lower in order of 415 nm, 460 nm, 540 nm, 600 nm, and 630 nm. Thus, when signal intensity of pixels indicating mucosal surfaces is equivalent in these pieces pf narrow-band light, signal intensity of a pixel indicating a blood vessel in each narrow-band image and a depth of the blood vessel have a relationship corresponding to a wavelength of each band, as illustrated in FIG. 4. Note that in FIG. 4, a horizontal axis indicates a depth of a blood vessel and a horizontal axis indicates signal intensity of a pixel indicating the blood vessel. Also, narrow-band light of 630 nm is not absorbed much on a mucosal surface and the signal intensity thereof becomes substantially the same as that of a pixel indicating a mucosal surface. Thus, the signal intensity of the narrow-band light is omitted in FIG. 4.

As illustrated in FIG. 4, in vicinity of a surface layer, signal intensity of the narrow-band image of 415 nm becomes the lowest. However, narrow-band light of 415 nm is scattered significantly. Thus, as a depth becomes deeper, signal intensity becomes higher and a difference with signal intensity of the narrow-band image of the 460 nm becomes small. Also, in a middle layer to a deep layer which is not reached by the narrow-band light of 415 nm, when signal intensity of narrow-band images of 540 nm and 600 nm is compared, signal intensity of the narrow-band image of 540 nm is small relatively on a surface layer side but a difference in signal intensity between the two becomes smaller as a depth becomes deeper.

That is, in the surface layer to the middle layer, an intensity ratio $I_{460}'/I_{415}'$ between the narrow-band images of 415 nm and 460 nm becomes higher as a depth becomes shallower. Thus, the intensity ratio $I_{460}'/I_{415}'$ can be used as depth feature data correlated to a depth in the surface layer to the middle layer. Also, in the middle layer to the deep layer, an intensity ratio $I_{540}'/I_{600}'$ between the narrow-band images of 600 nm and 540 nm becomes higher as a depth becomes deeper. Thus, the intensity ratio $I_{540}'/I_{600}'$ can be used as depth feature data correlated to a depth in the middle layer to the deep layer.

As detail processing, when the short-wavelength band selection unit 121a selects a narrow-band image on a short-wavelength side (such as narrow-band image of 415 nm) from the above-described five narrow-band images, the first feature data acquisition unit 121 acquires corrected signal intensity (such as intensity $I_{415}'$) of each pixel in the selected narrow-band image. Also, accordingly, the adjacent wavelength band selection unit 122a selects a narrow-band image (such as narrow-band image of 460 nm) a band of which is adjacent to that of the narrow-band image on the short-wavelength side and the second feature data acquisition unit 122 acquires corrected signal intensity (such as intensity $I_{460}'$) of each pixel in the selected narrow-band image. The ratio calculation unit 123 calculates, as depth feature data, a ratio $I_{460}'/I_{415}'$ of corrected signal intensity of pixels corresponding to each other in these narrow-band images.

Also, when the long-wavelength band selection unit 121b selects a narrow-band image on a long-wavelength side (such as narrow-band image of 600 nm) from the above-described five narrow-band images, the first feature data acquisition unit 121 acquires corrected signal intensity (such as intensity $I_{600}'$) of each pixel in the selected narrow-band image. Also, accordingly, the adjacent wavelength band selection unit 122a selects a narrow-band image (such as narrow-band image of 540 nm) a band of which is adjacent to that of the narrow-band image on the long-wavelength side and the second feature data acquisition unit 122 acquires corrected signal intensity (such as intensity $I_{540}'$) of each pixel in the selected narrow-band image. The ratio calculation unit 123 calculates, as depth feature data, a ratio $I_{540}'/I_{600}'$

of corrected signal intensity of pixels corresponding to each other in these narrow-band images.

Note that a combination of wavelengths to calculate an intensity ratio is not limited to the above-described combinations. For example, light absorption characteristics of the narrow-band light of 460 nm and the narrow-band light of 540 nm are relatively similar (see FIG. 4), an intensity ratio $I_{540}'/I_{415}'$ may be calculated instead of the intensity ratio $I_{460}'/I_{415}'$.

In next step S13, based on a ratio of the signal intensity (that is, depth feature data) calculated in step S12, the enhanced image creation unit 103 creates an enhanced image in which a blood vessel is highlighted in a color corresponding to a depth. The color corresponding to a depth is not specifically limited. In the first embodiment, a blood vessel in a surface layer is highlighted in yellow and a blood vessel in a deep layer is highlighted in blue. That is, in the created enhanced image, processing is performed in such a manner that a B component becomes smaller as a depth of a blood vessel becomes shallower and an R component becomes smaller as a depth of the blood vessel becomes deeper.

Here, narrow-band images of 460 nm, 540 nm, and 630 nm among the five narrow-band images acquired in step S10 are respectively approximate to a B component, a G component, and an R component of an image acquired with white light. Also, in the narrow-band image of 415 nm among the above five narrow-band images, signal intensity of a pixel indicating a blood vessel in a surface layer becomes lower than that of the other narrow-band images. On the other hand, in a narrow-band of 600 nm, signal intensity of a pixel indicating a blood vessel in a deep layer becomes lower than that of the other narrow-band images.

Thus, signal intensity of a B component in the enhanced image is calculated by adding the narrow-band image of 415 nm to the narrow-band image of 460 nm in such a manner that a ratio on a side of 415 nm becomes higher as a depth becomes shallower. On the other hand, signal intensity of an R component in the enhanced image is calculated by adding the narrow-band image of 600 nm to the narrow-band image of 630 nm in such a manner that a ratio on a side of 600 nm becomes higher as a depth becomes deeper. Accordingly, an image in which a blood vessel is highlighted according to a depth can be created.

Note that in the first embodiment, a blood vessel is highlighted according to a depth of a blood vessel. However, the blood vessel may be highlighted by contrast, chroma, luminance, or the like according to a depth of the blood vessel. For example, in a case of changing contrast according to a depth of a blood vessel, an image in which the blood vessel is highlighted while contrast being increased as a depth becomes shallower and contrast being decreased as a depth becomes deeper may be created. These examples are not the limitation. Based on information related to a depth of a blood vessel, various different methods to highlight the blood vessel can be applied.

FIG. 5 is a flowchart illustrating processing executed by the enhanced image creation unit 103 in step S13.

First, in step S131, the enhanced image creation unit 103 corrects intensity of the narrow-band image of 415 nm with respect to the narrow-band image of 460 nm. More specifically, by the following equation (1) using an AVG ($I_{630}/I_\lambda$) of the intensity ratio calculated in step S110, signal intensity of each pixel in the narrow-band image is corrected. In the equation (1), a sign $I_{415}''$ indicates signal intensity after correction is further performed on the corrected signal intensity $I_{415}'$.

$$I_{415}'' = \frac{I_{415}'}{AVG\left(\dfrac{I_{630}}{I_{460}}\right)} = I_{415} \times \frac{AVG\left(\dfrac{I_{630}}{I_{415}}\right)}{AVG\left(\dfrac{I_{630}}{I_{460}}\right)} \tag{1}$$

In next step S132, based on a ratio (intensity ratio) of signal intensity between narrow-band images, the enhanced image creation unit 103 calculates weight W1 and W2 given by the following equations (2) and (3). In the equations (2) and (3), signs $W1_{base}$ and $W2_{base}$ indicate the minimum values previously-set with respect to the weight W1 and W2 and signs $\alpha$ and $\beta$ ($\alpha$, $\beta > 0$) indicate parameters to control weight according to a ratio of signal intensity of narrow-band images.

$$W1 = W1_{base} + \alpha \times \left(\frac{I_{460}}{I_{415}}\right) \tag{2}$$

$$W2 = W2_{base} + \beta \times \left( \frac{I_{540}}{I_{600}} \right) \qquad (3)$$

According to the equation (2), the weight W1 becomes larger as a depth of a blood vessel becomes shallower. On the other hand, according to the equation (3), the weight W2 becomes larger as a depth of a blood vessel becomes deeper.

In the next step S133, the enhanced image creation unit 103 adds narrow-band images based on the weight W1 and W2. That is, signal intensity $I_B$, $I_G$, and $I_R$ of a B component, a G component, and an R component given by the following equations (4) to (6) is calculated and an image in which the signal intensity $I_B$, $I_G$, and $I_R$ is a pixel value is created.

$$I_B = W1 \times I_{415}'' + (1 - W1) \times I_{460} \qquad (4)$$

$$I_G = I_{540} \qquad (5)$$

$$I_R = W2 \times I_{600}' + (1 - W2) \times I_{630} \qquad (6)$$

As described above, the weight W1 becomes larger as a depth of a blood vessel becomes shallower. Thus, when a depth of the blood vessel is shallow, a ratio of the signal intensity $I_{415}''$ of the corrected narrow-band image of 415 nm in the signal intensity of the B component is increased and a value of the B component is controlled (that is, yellow become stronger). On the other hand, the weight W2 becomes larger as a depth of a blood vessel becomes deeper. Thus, when a depth of the blood vessel is deep, a ratio of the signal intensity $I_{600}'$ of the normalized narrow-band image of 600 nm in the signal intensity of the R component is increased and a value of the R component is controlled (that is, blue become stronger). Then, an operation of the image processing apparatus 1 goes back to a main routine.

In step S14 following step S13, the computing unit 100 outputs the enhanced image created in step S13, displays the image on the display unit 40, and records the image into the recording unit 50. Then, the processing in the image processing apparatus 1 is ended.

As described above, according to the first embodiment of the present invention, depth feature data correlated to a depth of a blood vessel is calculated based on signal intensity of at least three narrow-band images having different center wavelengths and the narrow-band images are added to one another based on the depth feature data. Thus, an image in which a blood vessel is highlighted in a color corresponding to a depth of the blood vessel can be created. Thus, by observing such an image, a user can observe a blood vessel in an intended depth in detail.

(Modification Example)

Next, a modification example of the first embodiment of the present invention will be described.

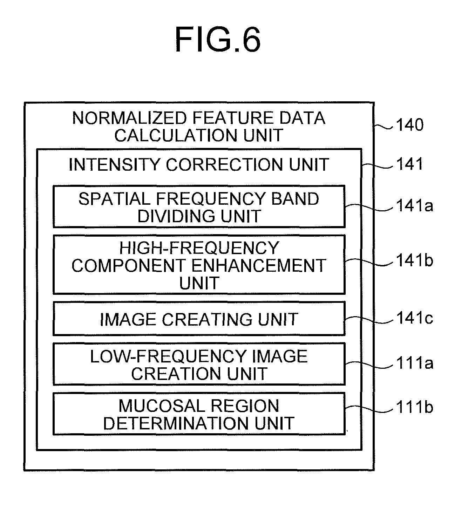

An image processing apparatus according to the modification example includes a normalized feature data calculation unit 140 illustrated in FIG. 6 instead of the normalized feature data calculation unit 110 in the image processing apparatus 1 illustrated in FIG. 1. Note that a configuration and an operation of each part other than the normalized feature data calculation unit 140 in the image processing apparatus according to the modification example are similar to those of the first embodiment.

As illustrated in FIG. 6, the normalized feature, data calculation unit 140 includes an intensity correction unit 141 to enhance signal intensity (hereinafter, also referred to as blood vessel signal) of a pixel, which indicates a blood vessel in each narrow-band image acquired by a narrow-band image acquisition unit 101 (see FIG. 1), according to a thickness of the blood vessel and to correct signal intensity of each pixel with respect to the enhanced narrow-band image.

More specifically, the intensity correction unit 141 further includes a spatial frequency band dividing unit 141a, a high-frequency component enhancement unit 141b, and an image creating unit 141c in addition to a low-frequency image creation unit 111a and a mucosal region determination unit 111b. Note that an operation of each of the low-frequency image creation unit 111a and the mucosal region determination unit 111b is similar to that of the first embodiment.

By performing spatial frequency resolution on each narrow-band image acquired by the narrow-band image acquisition unit 101, the spatial frequency band dividing unit 141a performs division into a plurality of spatial frequency bands. The high-frequency component enhancement unit 141b performs enhancement processing on each frequency component of the plurality of spatial frequency bands such that each frequency component is more enhanced as the frequency becomes higher. Based on the frequency component enhanced by the high-frequency component enhancement unit 141b, the image creating unit 141c creates a narrow-band image.

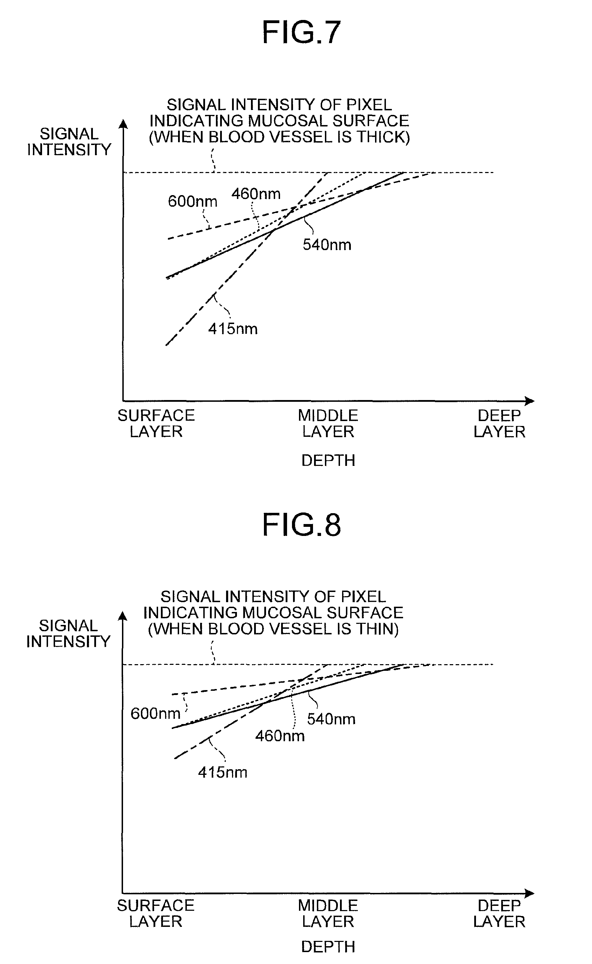

Here, as described above, intensity of a blood vessel signal in the narrow-band image and a depth of a blood vessel have characteristics corresponding to a wavelength of narrow-band light (see FIG. 4). Strictly speaking, these characteristics vary according to a thickness of the blood vessel. For example, as illustrated in FIG. 8, when a blood vessel is thin, absorption of narrow-band light is decreased as a whole. Thus, an intensity characteristic of the blood vessel signal as a whole is shifted to an upper side of a graph compared to a case, illustrated in FIG. 7, where a blood vessel is thick. In this case, even when depths of blood vessels are substantially the same, an intensity ratio (such as intensity ratio $I_{460}/I_{415}$ or $I_{540}/I_{600}$) between narrow-band images tends to be higher in a narrow blood vessel than in a thick blood vessel. Thus, in the modification example, by enhancing signal intensity of a pixel indicating a narrow blood vessel before calculating depth feature data, an influence due to a difference in light absorption corresponding to a thickness of a blood vessel is reduced.

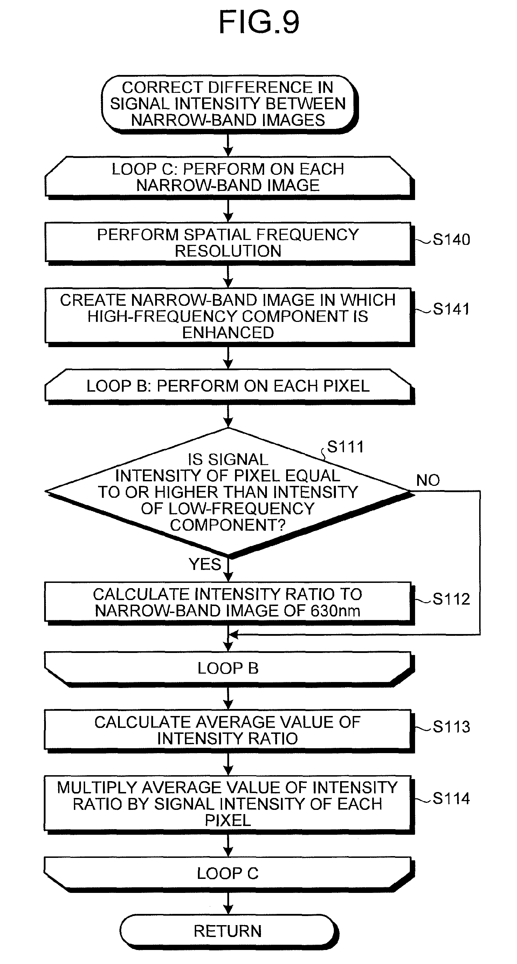

FIG. 9 is a flowchart illustrating processing executed by the normalized feature data calculation unit 140. Note that an operation of the whole image processing apparatus according to the modification example is similar to that of the first embodiment and only a detail operation in step S11 (see FIG. 2) executed by the normalized feature data calculation unit 140 is different from that of the first embodiment.

As illustrated in FIG. 9, the normalized feature data calculation unit 140 performs processing in a loop C on narrow-band images other than a reference narrow-band image (such as narrow-band image of 630 nm) among narrow-band images acquired by the narrow-band image acquisition unit 101.

First, in step S140, the spatial frequency band dividing unit 141a performs spatial frequency resolution on a narrow-band image as a processing target to divide into a plurality of spatial frequency bands. As a method of spatial frequency resolution, for example, DOG or the like described in the first embodiment can be used.

In next step S141, the high-frequency component enhancement unit 141b multiplies a coefficient by intensity of a component of each spatial frequency band divided by the spatial frequency band dividing unit 141a. Here, the higher the frequency band, the larger the coefficient is. Then, the image creating unit 141c adds up intensity of spatial frequency bands. In such a manner, a narrow-band image in which a high-frequency component is enhanced is created.

Then, based on the narrow-band image in which a high-frequency component is enhanced, steps S111 to S114 are executed. Note that processing in steps S111 to S114 is similar to that of the first embodiment. However, in and after step S111, processing is performed on a narrow-band image in which a high-frequency component is enhanced.

(Second Embodiment)

Next, a second embodiment of the present invention will be described.

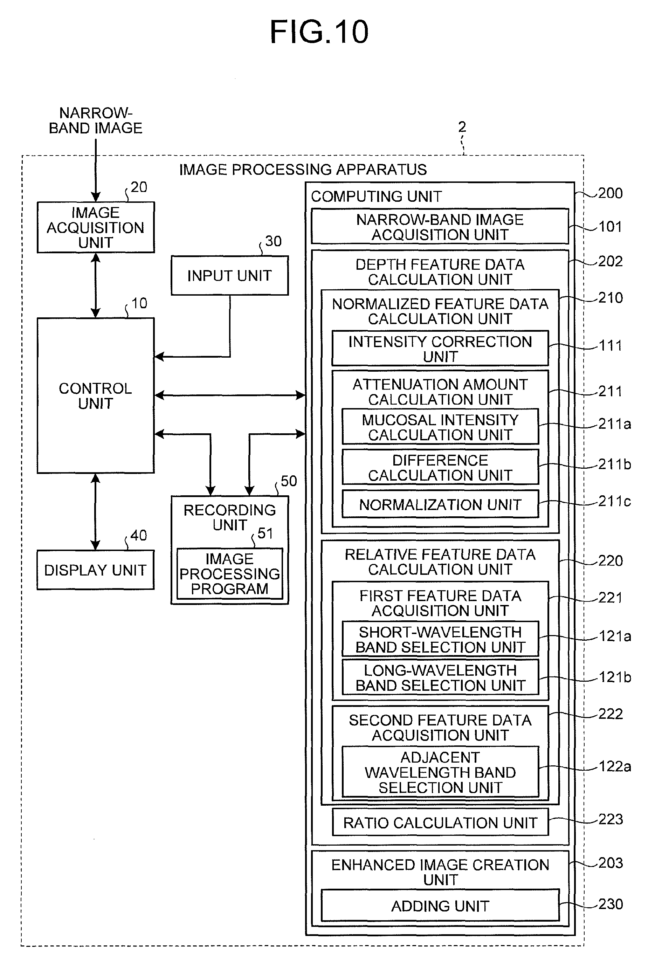

FIG. 10 is a block diagram illustrating a configuration of an image processing apparatus according to a second embodiment of the present invention. As illustrated in FIG. 10, the image processing apparatus 2 according to the second embodiment includes a computing unit 200 instead of the computing unit 100 illustrated in FIG. 1. A configuration and an operation of each part of the image processing apparatus 2 other than the computing unit 200 are similar to those of the first embodiment.

The computing unit 200 includes a narrow-band image acquisition unit 101, a depth feature data calculation unit 202, and an enhanced image creation unit 203. Here, an operation of the narrow-band image acquisition unit 101 is similar to that of the first embodiment.

The depth feature data calculation unit 202 includes a normalized feature data calculation unit 210 and a relative feature data calculation unit 220 and calculates depth feature data based on a narrow-band image acquired by the narrow-band image acquisition unit 101.

The normalized feature data calculation unit 210 further includes, in addition to an intensity correction unit 111, an attenuation amount calculation unit 211 to calculate an attenuation amount, due to light absorption of a wavelength component by a living body, of each narrow-band image acquired by the narrow-band image acquisition unit 101. Based on the attenuation amount, the normalized feature data calculation unit 210 normalizes signal intensity of each narrow-band image. Note that a configuration and an operation of the intensity correction unit 111 are similar to those of the first embodiment.

The attenuation amount calculation unit 211 includes a mucosal intensity calculation unit 211a, a difference calculation unit 211b, and a normalization unit 211c. Here, the mucosal intensity calculation unit 211a calculates signal intensity (hereinafter, also referred to as mucosal intensity) of a pixel indicating a mucosal surface among pixels included in each narrow-band image. More specifically, the mucosal intensity calculation unit 211a calculates, with respect to a narrow-band image, a low-frequency image in which a pixel value is a low-frequency component of a spatial frequency component. A pixel value of each pixel of a low-frequency image corresponds to mucosal intensity. Alternatively, a pixel value of each pixel in a long-wavelength band image including a wavelength component which is not absorbed much by hemoglobin may be used as mucosal intensity. Also, the difference calculation unit 211b calculates a difference with respect to mucosal intensity of signal intensity of each pixel included in each narrow-band image. Based on the mucosal intensity, the normalization unit 211c normalizes the difference.

The relative feature data calculation unit 220 includes a first feature data acquisition unit 221, a second feature data acquisition unit 222, and a ratio calculation unit 223. The first feature data acquisition unit 221 selects one narrow-band image (first narrow-band image) from the narrow-band images acquired by the narrow-band image acquisition unit 101 and acquires, as first feature data, a normalized difference which is calculated with respect to the selected narrow-band image. Based on a wavelength component of the narrow-band image selected by the first feature data acquisition unit 221, the second feature data acquisition unit 222 selects a different narrow-band image (second narrow-band image) from the narrow-band images acquired by the narrow-band image acquisition unit 101 and acquires, as second feature data, a normalized difference calculated with respect to the selected narrow-band image. Note that an operation of each of a short-wavelength band selection unit 121a and a long-wavelength band selection unit 121b included in the first feature data acquisition unit 221 and that of an adjacent wavelength band selection unit 122a included in the second feature data acquisition unit 222 are similar to those of the first embodiment. The ratio calculation unit 223 calculates a ratio between the first feature data and the second feature data as feature data indicating a relative attenuation amount between narrow-band images.

The enhanced image creation unit 203 includes an adding unit 230 for adding narrow-band images to one another. Based on the depth feature data calculated by the depth feature data calculation unit 202, the enhanced image creation unit 203 weights and adds the narrow-band image acquired by the narrow-band image acquisition unit 101 and the narrow-band image corrected by the intensity correction unit 111, and thereby creates an image in which a blood vessel is highlighted in a color corresponding to the depth.

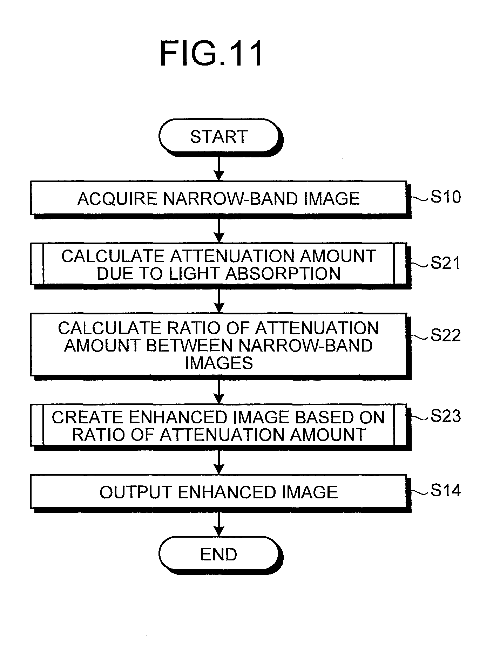

Next, an operation of the image processing apparatus 2 will be described. FIG. 11 is a flowchart illustrating an operation of the image processing apparatus 2. Note that an operation in each of steps S10 and S14 illustrated in FIG. 11 is similar to that of the first embodiment. Also, similarly to the first embodiment, in the second embodiment, five narrow-band images captured with pieces of narrow-band light centers of which are at 415 nm, 460 nm, 540 nm, 600 nm, and 630 nm are acquired as narrow-band images and image processing is performed.

In step S21 following step S10, the normalized feature data calculation unit 210 calculates an attenuation amount due to light absorption in each narrow-band image. Here, as described above, absorption of narrow-band light having a center wavelength of 630 nm by hemoglobin is significantly low. Thus, it is possible to consider that signal intensity of each pixel in the narrow-band image roughly indicates a mucosal surface. Thus, in the second embodiment, after correction is performed with a narrow-band image having a center wavelength 630 nm as a reference in such a manner that signal intensity of pixels indicating mucosal surfaces in the four other narrow-band images becomes equivalent, a difference in signal intensity with respect to the narrow-band image of 630 nm is calculated, whereby an attenuation amount is calculated.

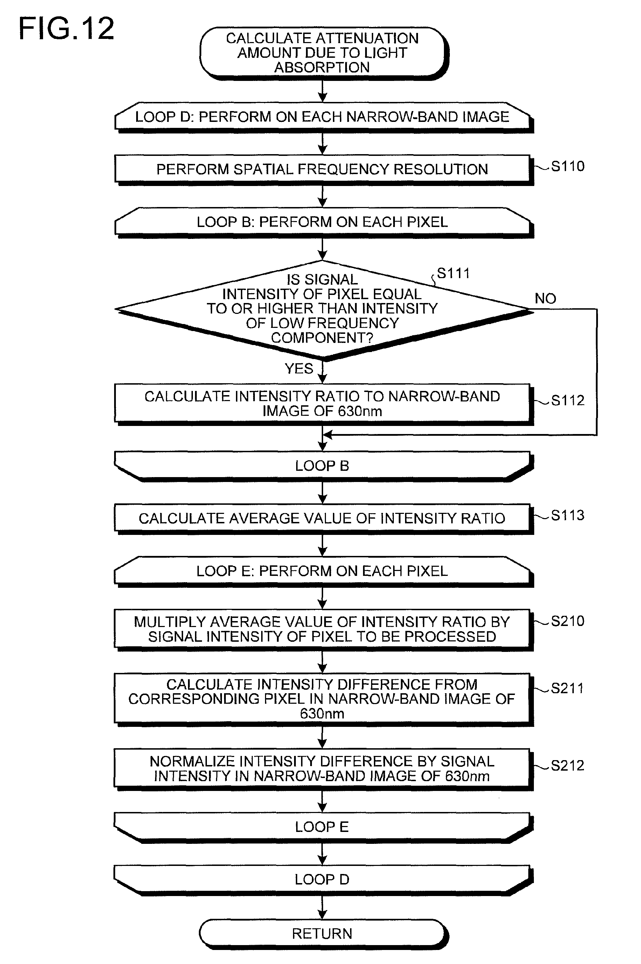

FIG. 12 is a flowchart illustrating processing executed by the normalized feature data calculation unit 210. The normalized feature data calculation unit 210 performs processing in a loop D on each narrow-band image acquired by the narrow-band image acquisition unit 101. Here, processing in steps S110 to S113 is similar to that of the first embodiment.

After step S113, the attenuation amount calculation unit 211 performs processing in a loop E on each pixel in the narrow-band images.

First, in step S210, the mucosal intensity calculation unit 211a multiplies an average value AVG $(I_{630}/I_\lambda)$ of an intensity ratio of a pixel indicating a mucosal surface calculated in step S113 by signal intensity $I_\lambda$ of a pixel as a processing target. Accordingly, signal intensity $I_\lambda$" which is the signal intensity $I_\lambda$ being corrected according to mucosal intensity is acquired.

In next step S211, the difference calculation unit 211b calculates a difference (intensity difference) $\Delta I_\lambda = I_\lambda \times$ AVG $(I_{630}/I_\lambda)$ - $I_{630}$ between the signal intensity $I_\lambda$" = $I_\lambda \times$ AVG $(I_{630}/I_\lambda)$ corrected in step S210 and signal intensity (that is, mucosal intensity) of a pixel in the narrow-band image of 630 nm corresponding to a pixel as a processing target.

In next step S212, by performing division by the signal intensity of the narrow-band image of 630 nm, the normalization unit 211c normalizes the difference $\Delta I$ (see next equation). This is because the intensity difference is a value which depends on intensity of a pixel indicating a mucosal surface. The normalized difference is used as an attenuation amount $A_\lambda$ ($\lambda$ = 415 nm, 460 nm, 540 nm, or 600 nm) in each narrow-band image. That is, $A_\lambda = \Delta I_\lambda/I_{630} = \{I_\lambda \times$ AVG $(I_{630}/I_\lambda)$ - $I_{630}\}/I_{630}$.

Note that in the second embodiment, the attenuation amount $A_\lambda$ is calculated with the narrow-band image of 630 nm as a reference but an attenuation amount may be calculated by a different method. For example, by assuming that a low-frequency component of each narrow-band image is a mucosal surface and normalizing signal intensity of each pixel with intensity of the low-frequency component in each narrow-band image as a reference (mucosal intensity), a difference between the normalized signal intensity and signal intensity of the low-frequency component may be calculated as an attenuation amount. Then, an operation of the image processing apparatus 2 will go back to a main routine.

In step S22 following step S21, the relative feature data calculation unit 220 calculates a ratio of the attenuation amount $A_\lambda$ calculated in step S21 between the narrow-band images different from one another. Here, as described above, a relationship between signal intensity of a pixel, which indicates a blood vessel in each narrow-band image, and a depth of the blood vessel corresponds to a wavelength in each band. Also, the attenuation amount calculated in step S21 is a difference in intensity of each piece of narrow-band light with respect to signal intensity of a pixel indicating the mucosal surface illustrated in FIG. 4. Thus, from a surface layer to a middle layer, a ratio $A_{460}/A_{415}$ between attenuation amounts of the narrow-band images of 415 nm and 460 nm becomes higher as a depth becomes shallower. On the other hand, from the middle layer to the deep layer, a ratio $A_{540}/A_{600}$ between attenuation amounts of the narrow-band images of 600 nm and 540 nm becomes higher as a depth becomes deeper.

Thus, in step S22, a ratio between the attenuation amounts is calculated as depth feature data correlated to a depth of a blood vessel in a living body. That is, the ratio $A_{460}/A_{415}$ between the attenuation amounts is used as depth feature data correlated to a depth in the surface layer to the middle layer and the ratio $A_{540}/A_{600}$ between the attenuation amounts is used as depth feature data correlated to a depth in the middle layer to the deep layer.

As detail processing, when the short-wavelength band selection unit 121a selects a narrow-band image on a short-wavelength side (such as narrow-band image of 415 nm) from the above-described five narrow-band images, the first feature data acquisition unit 221 acquires a corrected attenuation amount (such as attenuation amount $A_{415}$) of each pixel in the selected narrow-band image. Also, accordingly, the adjacent wavelength band selection unit 122a selects a narrow-band image (such as narrow-band image of 460 nm) a band of which is adjacent to that of the narrow-band image on the short-wavelength side and the second feature data acquisition unit 222 acquires a corrected attenuation amount (such as attenuation amount $A_{460}$) of each pixel in the selected narrow-band image. The ratio calculation unit 223 calculates, as depth feature data, a ratio $A_{460}/A_{415}$ between attenuation amounts of pixels corresponding to each other between the narrow-band images.

Also, when the long-wavelength band selection unit 121b selects a narrow-band image on a long-wavelength side (such as narrow-band image of 600 nm) from the above-described five narrow-band images, the first feature data acquisition unit 221 acquires a corrected attenuation amount (such as attenuation amount $A_{600}$) of each pixel in the selected narrow-band image. Also, accordingly, the adjacent wavelength band selection unit 122a selects a narrow-band image (such as narrow-band image of 540 nm) a band of which is adjacent to that of the narrow-band image on the long-wavelength side and the second feature data acquisition unit 222 acquires a corrected attenuation amount (such as attenuation amount $A_{540}$) of each pixel in the selected narrow-band image. The ratio calculation unit 223 calculates, as depth feature data, a ratio $A_{540}/A_{600}$ between attenuation amounts of pixels corresponding to each other in the narrow-band images.

Note that in the modification example of the first embodiment, it has been described that signal intensity in a narrow-band image varies depending on a thickness of a blood vessel. However, as a ratio between attenuation amounts used in the second embodiment, a variation of signal intensity due to a difference between thicknesses of blood vessels is canceled in a denominator and a numerator. Thus, depth feature data which does not depend on a thickness of a blood vessel can be acquired.

In next step S23, based on the depth feature data calculated in step S22, the enhanced image creation unit 203 creates an enhanced image in which a blood vessel is highlighted in a color corresponding to a depth. Similarly to the first embodiment, a blood vessel in a surface layer is highlighted in yellow and a blood vessel in a deep layer is highlighted in blue in the second embodiment.

A detail of processing in step S23 as a whole is similar to that of the first embodiment (see FIG. 5) but the following point is different. That is, the weight W1 and W2 is calculated based on signal intensity in the first embodiment (see step S132) but weight W1' and W2' is calculated based on an attenuation amount given by the following equations (7) and (8) in the second embodiment.

$$W1' = W1_{base} + \alpha \times \frac{A_{415}}{A_{460}} \qquad (7)$$

$$W2' = W2_{base} + \beta \times \frac{A_{600}}{A_{540}} \qquad (8)$$

In this case, in step S133, in the above-described equations (4) to (6), the weight W1' and W2' is used instead of the weight W1 and W2 and signal intensity $I_B$, $I_G$, and $I_R$ of a B component, a G component, and an R component is calculated.

As described above, according to the second embodiment, depth feature data correlated to a depth of a blood vessel is calculated based on attenuation amounts of pieces of narrow-band light calculated from at least three narrow-band images having different center wavelengths and the narrow-band images are added to one another based on the depth feature data. Thus, an image in which a blood vessel is highlighted in a color corresponding to a depth of the blood vessel can be created. Thus, by observing such an image, a user can observe a blood vessel in an intended depth in detail.

An image processing apparatus according to each of the above-described first embodiment, second embodiment, and modification example can be realized by executing an image processing program, which is recorded in a recording apparatus, with a computer system such as a personal computer or a work station. Also, such a computer system may be used by being connected to a device such as a different computer or a server through a local area network (LAN), a wide area network (WAN), or a public line such as the Internet. In this case, the image processing apparatus according to each of the first embodiment, second embodiment, and modification example may acquire image data of an intraluminal image through these networks, may output an image processing result to various output devices (such as viewer and printer) connected through these networks, or may store an image processing result into a storage apparatus (recording apparatus and reading apparatus thereof) connected through these networks.

Note that the present invention is not limited to the first embodiment, the second embodiment, and the modification example. By arbitrarily combining a plurality of elements disclosed in the embodiments and the modification example, various inventions can be formed. For example, a several elements may be removed from all elements described in the embodiments and the modification example or elements described in the different embodiments or modification example may be combined.

Reference Signs List

    1, 2 image processing apparatus
    10 control unit
    20 image acquisition unit
    30 input unit
    40 display unit
    50 recording unit
    51 image processing program
    100, 200 computing unit
    101 narrow-band image acquisition unit
    102, 202 depth feature data calculation unit
    103, 203 enhanced image creation unit
    110, 140, 210 normalized feature data calculation unit
    111 intensity correction unit
    111a low-frequency image creation unit
    111b mucosal region determination unit
    120, 220 relative feature data calculation unit
    121, 221 first feature data acquisition unit
    121a short-wavelength band selection unit
    121b long-wavelength band selection unit
    122, 222 second feature data acquisition unit
    122a adjacent wavelength band selection unit
    123, 223 ratio calculation unit
    130, 230 adding unit
    141 intensity correction unit
    141a spatial frequency band dividing unit
    141b high-frequency component enhancement unit
    141c image creating unit
    211 attenuation amount calculation unit
    211a mucosal intensity calculation unit
    211b difference calculation unit
    211c normalization unit

**Claims**

1. An image processing apparatus for processing an image acquired by imaging a living body, the image processing apparatus comprising:

a narrow-band image acquisition unit configured to acquire at least three narrow-band images with different center wavelengths from one another;
a depth feature data calculation unit configured to calculate depth feature data which is feature data correlated to a depth of a blood vessel in the living body based on a difference, between the narrow-band images different from one another, in variation of signal intensity due to an absorption variation of light with which the living body is irradiated; and
an enhanced image creation unit configured to create, based on the depth feature data, an image in which the blood vessel is highlighted according to the depth of the blood vessel,
wherein the depth feature data calculation unit includes:

a normalized feature data calculation unit configured to calculate pieces of normalized feature data by normalizing a value corresponding to signal intensity of each pixel in the at least three narrow-band images; and
a relative feature data calculation unit configured to calculate relative feature data indicating a relative relationship in intensity between the pieces of normalized feature data in the narrow-band images different from one another.

2. The image processing apparatus according to claim 1, wherein the normalized feature data calculation unit includes an attenuation amount calculation unit configured to calculate, with respect to each of the narrow-band images, an attenuation amount due to absorption of light of a wavelength component corresponding to each of the narrow-band images.

3. The image processing apparatus according to claim 2, wherein the attenuation amount calculation unit includes:

a mucosal intensity calculation unit configured to calculate mucosal intensity which is signal intensity of a pixel indicating a mucosal surface among pixels included in each of the narrow-band images;
a difference calculation unit configured to calculate a difference between the mucosal intensity and signal intensity of each pixel included in each of the narrow-band images; and
a normalization unit configured to normalize the difference based on the mucosal intensity.

4. The image processing apparatus according to claim 3, wherein the mucosal intensity calculation unit is configured to calculate a low-frequency image having, as a pixel value, a low-frequency component among a plurality of spatial frequency components constituting each of the narrow-band images.

5. The image processing apparatus according to claim 3, wherein one of the at least three narrow-band images is a long-wavelength band image having a wavelength component where absorption of light by hemoglobin is small, and the mucosal intensity calculation unit is configured to correct, using the long-wavelength band image as a reference, the signal intensity in the other narrow-band images.

6. The image processing apparatus according to claim 1, wherein the normalized feature data calculation unit includes an intensity correction unit configured to correct the signal intensity of each of the narrow-band images using signal intensity of a pixel indicating a mucosal region in the at least three narrow-band images as a reference.

7. The image processing apparatus according to claim 6, wherein the intensity correction unit includes:

a low-frequency image calculation unit configured to calculate, with respect to each of the narrow-band images, a low-frequency image having, as a pixel value, a low-frequency component among spatial frequency components constituting each of the narrow-band images; and
a mucosal region identification unit configured to identify a mucosal region in each of the narrow-band images based on each of the narrow-band images and the low-frequency image.

8. The image processing apparatus according to claim 6, wherein the intensity correction unit is configured to enhance the signal intensity of a pixel indicating the blood vessel in each of the narrow-band images, according to a thickness of the blood vessel, and to correct the signal intensity of each of the narrow-band images in which the pixel indicating the blood vessel has been enhanced.

9. The image processing apparatus according to claim 8, wherein the intensity correction unit includes:

a spatial frequency band dividing unit configured to divide each of the narrow-band images into a plurality of spatial frequency components;
a high-frequency component enhancement unit configured to enhance the plurality of spatial frequency components such that the plurality of spatial frequency components is more enhanced as a frequency becomes higher; and
an image creating unit configured to create a narrow-band image based on the plurality of spatial frequency components enhanced by the high-frequency component enhancement unit.

10. The image processing apparatus according to claim 1, wherein the relative feature data calculation unit includes:

a first feature data acquisition unit configured to select a first narrow-band image from among the at least three narrow-band images and to acquire normalized feature data of the first narrow-band image as first feature data;
a second feature data acquisition unit configured to select a second narrow-band image, which is different from the first narrow-band image, from among the at least three narrow-band images based on a wavelength component included in the first narrow-band image, and to acquire normalized feature data of the second narrow-band image as second feature data; and
a relative feature data calculation unit configured to calculate feature data indicating a relative value between the first feature data and the second feature data.

11. The image processing apparatus according to claim 10, wherein
the first feature data acquisition unit includes a short wavelength band selection unit configured to select a narrow-band image having a wavelength component with a relatively short wavelength from among the at least three narrow-band images, and
the first feature data acquisition unit is configured to acquire the normalized feature data in the narrow-band image selected by the short wavelength band selection unit.

12. The image processing apparatus according to claim 10, wherein
the first feature data acquisition unit includes a long wavelength band selection unit configured to select a narrow-band image having a wavelength component with a relatively long wavelength from among the at least three wavelength components, and
the first feature data acquisition unit is configured to acquire the normalized feature data in the narrow-band image selected by the long wavelength band selection unit.

13. The image processing apparatus according to claim 10, wherein
the second feature data acquisition unit includes an adjacent wavelength band selection unit configured to select a narrow-band image, a band of a wavelength component of which is adjacent to that of the first narrow-band image, from among the at least three narrow-band images, and
the second feature data acquisition unit is configured to acquire the normalized feature data in the narrow-band image selected by the adjacent wavelength band selection unit.

14. The image processing apparatus according to claim 10, wherein the relative feature data calculation unit includes a ratio calculation unit configured to calculate a ratio between the first feature data and the second feature data.

15. The image processing apparatus according to claim 1, wherein the enhanced image creation unit is configured to create, based on the depth feature data, the image in which the blood vessel is highlighted in a color according to the depth of the blood vessel.

16. The image processing apparatus according to claim 1, wherein the at least three narrow-band images include at least a red band image, a green band image, and a blue band image, respectively.

17. The image processing apparatus according to claim 1, wherein the enhanced image creation unit includes an adding unit configured to add the narrow-band images to one another based on the depth feature data to calculate signal intensity of each of a red component, a green component, and a blue component in a color image.

**18.** An image processing method executed by an image processing apparatus for processing an image acquired by imaging a living body, the method comprising:

a narrow-band image acquisition step of acquiring at least three narrow-band images with different center wavelengths from one another;
a depth feature data calculation step of calculating depth feature data which is feature data correlated to a depth of a blood vessel in the living body based on a difference, between the narrow-band images different from one another, in variation of signal intensity due to an absorption variation of light with which the living body is irradiated; and
an enhanced image creation step of creating, based on the depth feature data, an image in which the blood vessel is highlighted according to the depth of the blood vessel,
wherein the depth feature data calculation step includes:

a normalized feature data calculation step of calculating pieces of normalized feature data by normalizing a value corresponding to signal intensity of each pixel in the at least three narrow-band images; and
a relative feature data calculation step of calculating relative feature data indicating a relative relationship in intensity between the pieces of normalized feature data in the narrow-band images different from one another.

**19.** An image processing program causing an image processing apparatus for processing an image acquired by imaging a living body, to execute:

a narrow-band image acquisition step of acquiring at least three narrow-band images with different center wavelengths from one another;
a depth feature data calculation step of calculating depth feature data which is feature data correlated to a depth of a blood vessel in the living body based on a difference, between the narrow-band images different from one another, in variation of signal intensity due to an absorption variation of light with which the living body is irradiated; and
an enhanced image creation step of creating, based on the depth feature data, an image in which the blood vessel is highlighted according to the depth of the blood vessel,
wherein the depth feature data calculation step includes:

a normalized feature data calculation step of calculating pieces of normalized feature data by normalizing a value corresponding to signal intensity of each pixel in the at least three narrow-band images; and
a relative feature data calculation step of calculating relative feature data indicating a relative relationship in intensity between the pieces of normalized feature data in the narrow-band images different from one another.

# FIG.1

NARROW-
BAND IMAGE

IMAGE PROCESSING APPARATUS — 1

IMAGE ACQUISITION UNIT — 20

INPUT UNIT — 30

CONTROL UNIT — 10

DISPLAY UNIT — 40

RECORDING UNIT — 50

IMAGE PROCESSING PROGRAM — 51

COMPUTING UNIT — 100

NARROW-BAND IMAGE ACQUISITION UNIT — 101

DEPTH FEATURE DATA CALCULATION UNIT — 102

NORMALIZED FEATURE DATA CALCULATION UNIT — 110

INTENSITY CORRECTION UNIT — 111

LOW-FREQUENCY IMAGE CREATION UNIT — 111a

MUCOSAL REGION DETERMINATION UNIT — 111b

RELATIVE FEATURE DATA CALCULATION UNIT — 120

FIRST FEATURE DATA ACQUISITION UNIT — 121

SHORT-WAVELENGTH BAND SELECTION UNIT — 121a

LONG-WAVELENGTH BAND SELECTION UNIT — 121b

SECOND FEATURE DATA ACQUISITION UNIT — 122

ADJACENT WAVELENGTH BAND SELECTION UNIT — 122a

RATIO CALCULATION UNIT — 123

ENHANCED IMAGE CREATION UNIT — 103

ADDING UNIT — 130

EP 2 962 623 A1

# FIG.2

START

ACQUIRE NARROW-BAND IMAGE ~S10

CORRECT DIFFERENCE IN SIGNAL
INTENSITY BETWEEN NARROW-BAND
IMAGES ~S11

CALCULATE RATIO OF SIGNAL
INTENSITY BETWEEN NARROW-BAND
IMAGES ~S12

CREATE ENHANCED IMAGE BASED ON
RATIO OF SIGNAL INTENSITY ~S13

OUTPUT ENHANCED IMAGE ~S14

END

18

# FIG.3

CORRECT DIFFERENCE IN
SIGNAL INTENSITY BETWEEN
NARROW-BAND IMAGES

LOOP A: PERFORM ON EACH
NARROW-BAND IMAGE

PERFORM SPATIAL FREQUENCY
RESOLUTION — S110

LOOP B: PERFORM ON EACH PIXEL

S111

IS SIGNAL
INTENSITY OF PIXEL EQUAL
TO OR HIGHER THAN INTENSITY
OF LOW-FREQUENCY
COMPONENT?

NO

YES

CALCULATE INTENSITY RATIO TO
NARROW-BAND IMAGE OF 630nm — S112

LOOP B

CALCULATE AVERAGE VALUE OF
INTENSITY RATIO — S113

MULTIPLY AVERAGE VALUE OF INTENSITY
RATIO BY SIGNAL INTENSITY OF EACH
PIXEL — S114

LOOP A

RETURN

# FIG.4

SIGNAL INTENSITY OF PIXEL
INDICATING MUCOSAL SURFACE

SIGNAL
INTENSITY

600nm   460nm

540nm

415nm

SURFACE
LAYER

MIDDLE
LAYER

DEEP
LAYER

DEPTH

# FIG.5

CREATE ENHANCED IMAGE
BASED ON RATIO OF SIGNAL
INTENSITY

CORRECT INTENSITY OF NARROW-BAND
IMAGE OF 415nm  — S131

CALCULATE WEIGHT W1 AND W2 BASED
ON INTENSITY RATIO  — S132

ADD NARROW-BAND IMAGE BASED ON
WEIGHT  — S133

RETURN

# FIG.6

```
┌─────────────────────────────────────────┐
│ NORMALIZED FEATURE DATA          ┌─ 140  │
│ CALCULATION UNIT                          │
│  ┌───────────────────────────────────┐   │
│  │ INTENSITY CORRECTION UNIT    ┌─141 │   │
│  │  ┌─────────────────────────┐      │   │
│  │  │ SPATIAL FREQUENCY BAND  ┌─141a  │   │
│  │  │ DIVIDING UNIT           │      │   │
│  │  └─────────────────────────┘      │   │
│  │  ┌─────────────────────────┐      │   │
│  │  │ HIGH-FREQUENCY          │      │   │
│  │  │ COMPONENT ENHANCEMENT  ┌─141b   │   │
│  │  │ UNIT                    │      │   │
│  │  └─────────────────────────┘      │   │
│  │  ┌─────────────────────────┐      │   │
│  │  │ IMAGE CREATING UNIT    ┌─141c   │   │
│  │  └─────────────────────────┘      │   │
│  │  ┌─────────────────────────┐      │   │
│  │  │ LOW-FREQUENCY IMAGE    ┌─111a   │   │
│  │  │ CREATION UNIT           │      │   │
│  │  └─────────────────────────┘      │   │
│  │  ┌─────────────────────────┐      │   │
│  │  │ MUCOSAL REGION         ┌─111b   │   │
│  │  │ DETERMINATION UNIT      │      │   │
│  │  └─────────────────────────┘      │   │
│  └───────────────────────────────────┘   │
└─────────────────────────────────────────┘
```

# FIG.7

SIGNAL
INTENSITY

SIGNAL INTENSITY OF PIXEL
INDICATING MUCOSAL SURFACE
(WHEN BLOOD VESSEL IS THICK)

600nm    460nm

540nm

415nm

SURFACE
LAYER

MIDDLE
LAYER

DEEP
LAYER

DEPTH

# FIG.8

SIGNAL
INTENSITY

SIGNAL INTENSITY OF PIXEL
INDICATING MUCOSAL SURFACE
(WHEN BLOOD VESSEL IS THIN)

600nm

540nm

460nm

415nm

SURFACE
LAYER

MIDDLE
LAYER

DEEP
LAYER

DEPTH

# FIG.9

CORRECT DIFFERENCE IN
SIGNAL INTENSITY BETWEEN
NARROW-BAND IMAGES

LOOP C: PERFORM ON EACH
NARROW-BAND IMAGE

PERFORM SPATIAL FREQUENCY
RESOLUTION — S140

CREATE NARROW-BAND IMAGE IN WHICH
HIGH-FREQUENCY COMPONENT IS
ENHANCED — S141

LOOP B: PERFORM ON EACH PIXEL

S111
IS SIGNAL
INTENSITY OF PIXEL EQUAL
TO OR HIGHER THAN INTENSITY
OF LOW-FREQUENCY
COMPONENT? — NO

YES

CALCULATE INTENSITY RATIO TO
NARROW-BAND IMAGE OF 630nm — S112

LOOP B

CALCULATE AVERAGE VALUE OF
INTENSITY RATIO — S113

MULTIPLY AVERAGE VALUE OF INTENSITY
RATIO BY SIGNAL INTENSITY OF EACH
PIXEL — S114

LOOP C

RETURN

# FIG.10

NARROW-
BAND IMAGE

2

IMAGE PROCESSING APPARATUS

20
IMAGE
ACQUISITION
UNIT

30
INPUT UNIT

10
CONTROL
UNIT

50
RECORDING
UNIT
51
IMAGE
PROCESSING
PROGRAM

40
DISPLAY UNIT

COMPUTING UNIT — 200

NARROW-BAND IMAGE
ACQUISITION UNIT — 101

DEPTH FEATURE DATA
CALCULATION UNIT — 202

NORMALIZED FEATURE DATA
CALCULATION UNIT — 210

INTENSITY CORRECTION
UNIT — 111

ATTENUATION AMOUNT
CALCULATION UNIT — 211

MUCOSAL INTENSITY
CALCULATION UNIT — 211a

DIFFERENCE
CALCULATION UNIT — 211b

NORMALIZATION UNIT — 211c

RELATIVE FEATURE DATA
CALCULATION UNIT — 220

FIRST FEATURE DATA
ACQUISITION UNIT — 221

SHORT-WAVELENGTH
BAND SELECTION UNIT — 121a

LONG-WAVELENGTH
BAND SELECTION UNIT — 121b

SECOND FEATURE DATA
ACQUISITION UNIT — 222

ADJACENT
WAVELENGTH BAND
SELECTION UNIT — 122a

RATIO CALCULATION UNIT — 223

ENHANCED IMAGE CREATION
UNIT — 203

ADDING UNIT — 230

24

# FIG.11

```
              ┌──────────┐
              │  START   │
              └──────────┘
                   │
                   ▼
    ┌───────────────────────────────────┐
    │   ACQUIRE NARROW-BAND IMAGE        │──S10
    └───────────────────────────────────┘
                   │
                   ▼
    ┌───────────────────────────────────┐
    │  CALCULATE ATTENUATION AMOUNT      │──S21
    │     DUE TO LIGHT ABSORPTION        │
    └───────────────────────────────────┘
                   │
                   ▼
    ┌───────────────────────────────────┐
    │  CALCULATE RATIO OF ATTENUATION    │──S22
    │  AMOUNT BETWEEN NARROW-BAND        │
    │            IMAGES                  │
    └───────────────────────────────────┘
                   │
                   ▼
    ┌───────────────────────────────────┐
    │ CREATE ENHANCED IMAGE BASED ON     │──S23
    │   RATIO OF ATTENUATION AMOUNT      │
    └───────────────────────────────────┘
                   │
                   ▼
    ┌───────────────────────────────────┐
    │     OUTPUT ENHANCED IMAGE          │──S14
    └───────────────────────────────────┘
                   │
                   ▼
              ┌──────────┐
              │   END    │
              └──────────┘
```

# FIG.12

```
┌─────────────────────────────┐
│   CALCULATE ATTENUATION     │
│   AMOUNT DUE TO LIGHT       │
│        ABSORPTION           │
└─────────────────────────────┘
              │
              ▼
╱─────────────────────────────────────────────╲
│  LOOP D: PERFORM ON EACH NARROW-BAND IMAGE    │
╲─────────────────────────────────────────────╱
              │
              ▼
┌─────────────────────────────────────┐
│  PERFORM SPATIAL FREQUENCY RESOLUTION │──S110
└─────────────────────────────────────┘
              │
              ▼
╱─────────────────────────────────────╲
│     LOOP B: PERFORM ON EACH PIXEL     │
╲─────────────────────────────────────╱
              │
              ▼
        ╱─────────────────╲              S111
       ╱    IS SIGNAL      ╲
      ╱  INTENSITY OF PIXEL  ╲
     ╱  EQUAL TO OR HIGHER    ╲    NO
    ╲  THAN INTENSITY OF LOW   ╱──────────┐
     ╲  FREQUENCY COMPONENT?  ╱           │
      ╲───────────────────────╱           │
              │ YES                        │
              ▼                            │
┌─────────────────────────────────┐       │
│ CALCULATE INTENSITY RATIO TO     │──S112 │
│ NARROW-BAND IMAGE OF 630nm       │       │
└─────────────────────────────────┘       │
              │◄──────────────────────────┘
              ▼
╱─────────────────────────────────────╲
│              LOOP B                    │
╲─────────────────────────────────────╱
              │
              ▼
┌─────────────────────────────────────┐
│ CALCULATE AVERAGE VALUE OF INTENSITY  │──S113
│              RATIO                    │
└─────────────────────────────────────┘
              │
              ▼
╱─────────────────────────────────────╲
│     LOOP E: PERFORM ON EACH PIXEL     │
╲─────────────────────────────────────╱
              │
              ▼
┌─────────────────────────────────────┐
│ MULTIPLY AVERAGE VALUE OF INTENSITY   │──S210
│ RATIO BY SIGNAL INTENSITY OF PIXEL    │
│         TO BE PROCESSED               │
└─────────────────────────────────────┘
              │
              ▼
┌─────────────────────────────────────┐
│ CALCULATE INTENSITY DIFFERENCE FROM   │──S211
│ CORRESPONDING PIXEL IN NARROW-BAND    │
│         IMAGE OF 630nm                │
└─────────────────────────────────────┘
              │
              ▼
┌─────────────────────────────────────┐
│ NORMALIZE INTENSITY DIFFERENCE BY     │──S212
│ SIGNAL INTENSITY IN NARROW-BAND       │
│         IMAGE OF 630nm                │
└─────────────────────────────────────┘
              │
              ▼
╱─────────────────────────────────────╲
│              LOOP E                    │
╲─────────────────────────────────────╱
              │
              ▼
╱─────────────────────────────────────╲
│              LOOP D                    │
╲─────────────────────────────────────╱
              │
              ▼
        ┌─────────────┐
        │   RETURN    │
        └─────────────┘
```

| | INTERNATIONAL SEARCH REPORT | International application No. |
|---|---|---|
| | | PCT/JP2014/050772 |

A. CLASSIFICATION OF SUBJECT MATTER
*A61B1/04*(2006.01)i, *A61B1/00*(2006.01)i, *A61B1/06*(2006.01)i, *H04N7/18*
(2006.01)i

According to International Patent Classification (IPC) or to both national classification and IPC

B. FIELDS SEARCHED

Minimum documentation searched (classification system followed by classification symbols)
A61B1/04, A61B1/00, A61B1/06, H04N7/18

Documentation searched other than minimum documentation to the extent that such documents are included in the fields searched
Jitsuyo Shinan Koho        1922–1996    Jitsuyo Shinan Toroku Koho    1996–2014
Kokai Jitsuyo Shinan Koho   1971–2014    Toroku Jitsuyo Shinan Koho    1994–2014

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2012-90725 A (Fujifilm Corp.),<br>17 May 2012 (17.05.2012),<br>entire text; all drawings<br>& US 2012/0101348 A1    & EP 2446809 A1 | 1–19 |
| A | JP 2011-218135 A (Fujifilm Corp.),<br>04 November 2011 (04.11.2011),<br>entire text; all drawings<br>& US 2011/0077462 A1    & EP 2305094 A1 | 1–19 |
| A | JP 2012-66066 A (Fujifilm Corp.),<br>05 April 2012 (05.04.2012),<br>entire text; all drawings<br>& JP 2012-66065 A        & US 2012/0053434 A1 | 1–19 |

☐ Further documents are listed in the continuation of Box C.     ☐ See patent family annex.

| * | Special categories of cited documents: |
|---|---|
| "A" | document defining the general state of the art which is not considered   to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>    26 March, 2014 (26.03.14) | Date of mailing of the international search report<br>    08 April, 2014 (08.04.14) |
|---|---|
| Name and mailing address of the ISA/<br>    Japanese Patent Office | Authorized officer |
| Facsimile No. | Telephone No. |

Form PCT/ISA/210 (second sheet) (July 2009)

27

**EP 2 962 623 A1**

**Patent documents cited in the description**

- JP 2011098088 A